# EUROPÄISCHE PATENTANMELDUNG

(11) **EP 1 190 640 A2**
(43) Veröffentlichungstag der Anmeldung: **27.03.2002**
(21) Anmeldenummer: 01122355.9
(22) Anmeldetag: 19.09.2001
(51) Int. Cl.: A45D 34/04

(54) **Drehbarer Applikator mit einer strukturierten Oberfläche**

(30) Priorität: 21.09.2000 DE 10047448
(71) Anmelder: Beiersdorf AG, 20245 Hamburg (DE)
(72) Erfinder: Eckers, Lorenz, 21255 Tostedt (DE); Maurer, Peter, 22880 Wedel (DE); Kux, Ulrich, Dr., 22559 Hamburg (DE); Untiedt, Sven, Dr., 20259 Hamburg (DE); Teichmann, Stephan, Dr., 22800 Wedel (DE)

(57) **Zusammenfassung**

Applikator für Flüssigkeiten, fließfähige Formulierungen, Pasten, Pulver und dergleichen, bestehend aus einer Lagerung und einem in der Lagerung gehaltenen, rotationsfähigen geometrischen Körper, wobei der rotationsfähige geometrische Körper zumindest mit einem Teil seiner Oberfläche freiliegt, dadurch gekennzeichnet, daß der rotationsfähige geometrische Körper (1) eine strukturierte Oberfläche aufweist.

## Beschreibung

Die Erfindung betrifft einen Applikator für Flüssigkeiten, fließfähige Formulierungen, Pasten, Pulver und dergleichen, bestehend aus einer Lagerung und einem in der Lagerung gehaltenen, rotationsfähigen Körper sowie die Verwendung eines rotationsfähigen Körpers mit einer strukturierten Oberfläche zur Applikation von Substanzen auf ein Substrat.

Vorrichtungen, bei denen eine frei drehbare Kugel in einer Lagerung zur Auftragung von Flüssigkeiten auf ein Substrat verwendet werden, sind seit langem in großer Zahl bekannt. Solche Kugelauftragvorrichtungen sind häufig derart gestaltet, daß die Kugel auf einem ringförmigen Dichtungssitz aufliegt und dabei in einem Halterungs- oder Lagersitz eingeschlossen ist, welcher sich oberhalb des Dichtungssitzes befindet. Dabei ragt die Kugel mit einem Teil Ihrer Oberfläche aus dem Halterungssitz heraus, ein weiteres, dem herausragenden Teil gegenüberliegendes Teil der Oberfläche taucht in die abzugebende Flüssigkeit ein.
Wird die Kugel nun über ein Substrat gerollt, so haftet der Kugel ein Teil der Flüssigkeit als dünne Schicht auf der Oberfläche an und wird während der Drehbewegung nach außen transportiert, so daß die benetzte Oberfläche zum freiliegenden Kugelteil wird. Von hier aus kann die Flüssigkeit nun leicht während der Rollbewegung auf das Substrat aufgetragen werden.
Neben beispielsweise Kugelschreibern, bei denen Tinte über eine rollende Kugel auf die Schreibunterlage übertragen wird, dienen derlei Vorrichtungen in erster Linie für die Auftragung von Flüssigkeiten im kosmetischen und dermatologischen Bereich.

Bevorzugt werden derlei Kugelauftragvorrichtungen auf einer Flasche mit der aufzutragenden Flüssigkeit angebracht, wobei die Flasche mit einem Schraubdeckel verschlossen werden kann.

Bereits in der DE 11 76 802 A1 ist eine Kugelauftragvorrichtung für kosmetische Mittel und dergleichen für einen mit einer Öffnung versehenen Behälter, mit der ein federnd nachgiebiges, den Rand der Behälteröffnung überdeckendes Lagerstück im Eingriff steht, beschrieben, wobei das Lagerstück mit einem unteren ringförmigen Dichtungssitz versehen ist, gegen den eine Kugel, die von dem Lagerstück oberhalb des Sitzes eingeschlossen ist und zum Teil über das Lagerstück hinausragt, dadurch drückbar ist, daß der innere Deckelteil einer abnehmbar mit dem Behälter im Eingriff stehenden und das Lagerstück frei übergreifenden Kappe unmittelbar auf den aus dem Lagerstück herausragenden Teil der Kugel drückt. Die Kappe weist hierbei eine Schulter auf, die sich gegen eine Schulter des Lagerstücks legt.

Solch eine Auftragsvorrichtung ist in der DE 31 04 525 A1 modifiziert, diese Schrift erläutert eine Kugelauftragvorrichtung mit Schraubkappe für eine Flasche für kosmetische Mittel mit einem mit dem Rand der Flasche fest verbindbaren ringförmigen Lagerstück, welches einen unteren Dichtsitz und einen oberen Halterungssitz für eine Kugel aufweist. An der Schraubkappe und dem Lagerstück sind miteinander in Eingriff bringbare Rast-, Schnapp- oder Klemmverbindungsteile zur lösbaren Verbindung zwischen Lagerstück und Schraubkappe angeordnet.

Ein Problem bei Auftragsvorrichtungen wie den oben beschriebenen besteht darin, daß die Kugel austrocknen kann: Damit permanent Flüssigkeit an die Außenseite des Applikators transportiert werden kann, muß die Kugel in die Flüssigkeit eintauchen, dadurch ist bedingt, daß solche Auftragsvorrichtungen nicht in allen Stellungen funktionieren. bei sinkendem Flüssigkeitsspiegel oder bei Schrägstellung der Vorrichtung ist die Benetzung der Kugel nicht mehr gewährleistet und die Auftragsvorrichtung nicht mehr funktionsfähig.

Mit dem Problem der Förderung der Flüssigkeit unter die Kugel beschäftigt sich daher die DE 42 15 966 A1. Vorgestellt wird hier ein Kosmetikroller mit elastischem Behälter oder Behälterteil mit einem Auftragselement, als Kugel oder Walze ausgebildet und mit einem Lager, an dem ein bewegbares, schlauchartiges Fördermittel angeordnet ist, das ein- oder mehrteilig und/oder mit einem Massestück am Ende versehen ist, wobei ein Fitmenteinsatz mit integriertem Speicherraum mit innen axial verlaufenden Kapillaren und mit angeformtem Fördermittel oder mit schlauchartigem Fördermittel entweder in einem einteilig mit einem Fitment zusammen hergestellten Kosmetikrollerbehälter, in einem separaten Fitment oder in einem verlängerten Fitment angeordnet ist.

Auch die DE 198 27 965 beschäftigt sich mit der Lösung der Aufgabe, ein Benetzen der Kugel unabhängig von der Lage der Kugel zu gestatten. Hierzu wird eine Kugelauftragvorrichtung angeboten, bestehend aus einer Kugel, einem Kugelhalter und einem Vorratsbehälter, wobei (a) unter der Kugel wenigstens eine der Vorratskammern angeordnet ist, (b) die Vorratskammern über Öffnungen mit dem Flüssigkeitsbehälter verbunden sind, (c) eine der Wände der Vorratskammern durch einen Teil der Oberfläche der Kugel gebildet wird.

Die Ausbildung der Auftragskugel berücksichtigt die EP 0 792 635 A2, welche ein Antiperspirant beansprucht, welches in einen Vorratsbehälter verpackt ist, wobei der Vorratsbehälter eine Kugel mit einem Durchmesser von mindestens 3 cm besitzt, und wobei das Antiperspirant zusammengesetzt ist aus 10 bis 70 Gew.-% Ethanol, bis zu 1 % Verdickungsmittel, 5 bis 20 Gew.-% eines Antiperspirantwikrstoffes und bis zu 5 Gew.-% eines die Fließfähigkeit erhöhenden Mittels.

Nachteilig bei all den bisher geschilderten Systemen ist die begrenzte Benetzbarkeit der Auftragskugel bzw. deren mengenmäßig nur eingeschränkte Transportfähigkeit für Flüssigkeiten. Weiterhin nachteilig bei diesen Systemen ist, daß man die Kugel zur Benetzung der Oberfläche zunächst mechanisch "aktivieren" muß, wenn der Applikator des längeren nicht verwendet wurde: Durch die geringe Menge an Flüssigkeit auf der Außenseite der Kugel trocknet diese relativ schnell ein, so daß man die Kugel zunächst einige Male umdrehen muß, damit die Oberfläche wieder vollständig mit dem aufzutragenden Substrat benetzt ist.

Aufgabe der Erfindung ist es daher, einen Applikator anzubieten, welcher die genannten Nachteile des Standes der Technik nicht oder nur in vermindertem Maße aufweist. Dieser Applikator soll ein Auftragssystem besitzen, bei dem die Flüssigkeitsverfügbarkeit während des Auftragsvorganges erhöht ist. Die Wahrscheinlichkeit des Abreißens des Flüssigkeitsfilmes während des Auftragsvorganges soll ebenso verringert werden wie das Austrocknen des Applikators während der Phasen, in denen der Applikator nicht verwendet wird, es soll also eine sofortige Produktverfügbarkeit bestehen. Weiterhin soll der Applikator eine bessere Rollbarkeit aufweisen.

Gelöst wird diese Aufgabe überraschend durch einen Applikator, wie er im Hauptanspruch beschrieben wird. Hierbei wird als Auftragsvorrichtung ein rotationsfähiger geometrischer Körper (im folgenden als Auftragskörper bezeichnet) mit einer strukturierten Oberfläche eingesetzt. Die Unteransprüche betreffen vorteilhafte Ausführungsformen dieses Applikators. Beansprucht wird weiterhin die Verwendung eines solchen rotationsfähigen Körpers mit einer strukturierten Oberfläche zur Applikation von Substanzen auf ein Substrat.

Dementsprechend beschreibt der Hauptanspruch einen Applikator für Flüssigkeiten, fließfähige Formulierungen, Pasten, Pulver und dergleichen, bestehend aus einer Lagerung und einem in der Lagerung gehaltenen, rotationsfähigen geometrischen Körper, wobei der rotationsfähige geometrische Körper zumindest mit einem Teil seiner Oberfläche aus der Lagerung herausragt, und wobei der rotationsfähige geometrische Körper eine strukturierte Oberfläche aufweist.

Bevorzugt handelt es sich bei den fließfähigen Formulierungen um Emulsionen, Suspensionen, Kolloide, Dispersionen, Gele oder Lösungen.

In einer ersten Ausführungsform eines solchen erfinderischen Applikators ist der rotationsfähige Körper ein rotationssymmetrischer geometrischer Körper.

In einer sehr vorteilhaften weiteren Ausführungsform werden durch die Strukturierung Vertiefungen in der Oberfläche gebildet, insbesondere Vertiefungen in symmetrischer Anordnung. In einer weiteren Variante des erfinderischen Applikators ist der Auftragskörper strukturiert, indem sich Erhebungen auf der Oberfläche befinden.

Die Vertiefungen in der Oberfläche des Auftragskörpers liegen sehr bevorzugt in Form von Mulden, Rinnen, Kerben, Kanälen und dergleichen vor. Ohne Einschränkungen lassen sich hier aber alle Vertiefungen für den erfinderischen Applikator zur Erhöhung der Funktionalität nutzen, beispielsweise auch Vertiefungen in Form von Ornamenten, Figuren oder Schriftzeichen und dergleichen.

Für den erfinderischen Applikator sind die beiden im folgenden beschriebenen Varianten im erfinderischen Sinne sehr günstig: Zum einen können die Vertiefungen derart angeordnet und dimensioniert sein, daß die Vertiefungen separat voneinander vorliegen, beispielsweise in den oben erwähnten Mulden, wenn diese keinen Kontakt zueinander besitzen ("geschlossenporig"), zum anderen können die Vertiefungen untereinander in Verbindung stehen, so daß sich ein "Kanalsystem" in der makroskopischen Oberfläche der Kugel befindet ("offenporig; in einer altemativen Betrachtungsweise ist dies äquivalent zu den oben erwähnten Erhebungen auf der Kugeloberfläche, wenn der "Grund" der Kanäle als makroskopische Oberfläche der Kugel angesehen wird).
Durch die angebrachten Vertiefungen wird die transportierte Flüssigkeitsmenge erhöht. Zusätzlich zu der bekannten Benetzung der makroskopischen Oberfläche des Auftragskörpers sammelt sich die Flüssigkeit in den Vertiefungen wie in kleinen Töpfen und wird von diesen, ähnlich der Funktionsweise einer Schaufelradpumpe, an die Oberfläche transportiert.
Ebenso kann die abzugebende Substanz durch den strukturierten Auftragskörper bei der Aufbringung auf das Substrat deutlich besser dosiert werden.
Eine symmetrische Anordnung der Vertiefungen auf dem Auftragskörper führt im Vergleich zu einer unsymmetrischen Anordnung zu einer gleichmäßigeren Flüssigkeitsverteilung auf der Oberfläche und damit zu verbessertem Auftragsverhalten.

In einer weiteren verbesserten Ausführungsform des erfinderischen Applikators ist der Auftragskörper eine Kugel, ein Ellipsoid oder eine Walze. Derartige Formen sind mit makroskopisch glatten Oberflächen für auf dem Markt befindliche Applikatoren bekannt. Weitere gut geeignete Formen für den rotationsfähigen Auftragskörper des erfinderischen Applikators sind beispielsweise Kegel, Hyperboloide, Drehparaboloide sowie jeweils Ab- oder Ausschnitte oder Stümpfe dieser geometrischen Körper sowie unregelmäßige geometrische Körper. Letztere können zum Beispiel dazu geeignet sein, auf unebenen Substraten eine optimierte Auftragung zu erzielen.
Besonders vorteilhaft ist der erfinderische Applikator Teil eines den abzugebenden Stoff beinhaltenden Behälters, insbesondere eines flaschenartigen Behälters. Der erfinderische Applikator läßt sich dann als Auftragsvorrichtung für Darreichungssysteme für Flüssigkeiten verwenden, welche beispielsweise den vorbekannten Deo- bzw. Antitranspirant Rollem entspricht.

Die Lagerung besteht bevorzugt aus einem Kunststofffitment, bestehend aus einem Ring, dessen Form die Form des Auftragskörpers im wesentlichen aufnimmt und dessen Innendurchmesser geringförmig größer ist als der Außendurchmesser des Auftragskörpers (bei elliptischen Körpern sind die beiden Halbachsen entsprechend etwas größer als diejenigen der Auftragsvorrichtung).
Im weiteren wird dieses Fitment anhand einer kugelförmigen Auftragsvorrichtung (Kugel) erläutert, wobei für anders geformte Auftragskörper die Form des Fitments entsprechend der Form des Auftragskörpers anzupassen ist und die Beschreibung entsprechend übertragen werden kann.
Nach unten hin ist dieser Ring durch ein Auflagebett für die Kugel, welches zum Vorratsgefäß hin eine Öffnung aufweist, abgeschlossen, von dieser Seite aus wird die Kugel mit der aufzutragenden Substanz benetzt. Bevorzugt ist diese Seite des Fitments auf das die aufzutragende Substanz beinhaltende Vorratsgefäß aufgesetzt oder in dieses eingepaßt. In einer Variante sind das Fitment und das Vorratsgefäß einteilig gestaltet.
Nach oben hin verjüngt sich der Ring geringfügig, so daß der Durchmesser der verbleibenden Öffnung etwas kleiner ist als der Durchmesser der Kugel. Die Kugel kann nun mit leichtem Druck in das Fitment eingelegt werden, wobei sie in dieses "einschnappt" und nicht von selbst wieder herausfällt. Dabei ragt sie mit einem Teil ihrer Oberfläche aus dem Fitment heraus und ist in alle Richtungen frei drehbar, so daß durch eine Rollbewegung wie beschrieben die Substanz auf ein Substrat aufgetragen werden kann.
In einer Variante dieses Fitment ist kein vollständiger Ring vorhanden, sondern es sind zumindest zwei, bevorzugt mindestens drei Ringausschnitte vorhanden, um die Kugel zu halten. Diese sind entsprechend so ausgeformt, daß die Kugel nach dem Einschnappen nicht aus dem Fitment herausfällt. Diese Fitmentgestaltung bedarf allerdings zusätzlichen Aufwandes bei der Abdichtung zum Vorratsbehälter hin.
Im Falle einer walzenförmigen Auftragsvorrichtung besteht die Lagerung aus zwei gegenüberliegenden Wänden, welche nach unten hin ebenfalls durch ein Auflagebett mit einer Öffnung zum Vorratsgefäß und nach oben hin mit einer Verjüngung zur jeweils anderen Wand hin abgeschlossen werden. In einer bevorzugten Ausführungsform sind die zwei Seitenwände dabei Abschnitte eines Rohres, dessen Durchmesser etwas größer ist als der Durchmesser der Walze. Nach vorn und nach hinten kann dieser Röhrenausschnitt durch zwei planparallele Wände verschlossen werden.

Wählt man die Maße der erfinderischen oberflächenstrukturierten Kugel, des Ellipsoids oder der Walze entsprechend, so lassen sich die strukturierten Systeme in die bekannten Lagersysteme einsetzen. Diese Vorgehensweise erlaubt es, die bisherigen Fertigungsanlagen größtenteils weiterzuverwenden, was die finanziellen Aufwendungen zur Fertigung der erfinderischen Applikatoren erheblich vermindert.

In einer sehr bevorzugten Ausführungsform der Lagerung des erfinderischen Applikators ist diese derart mit der Öffnung eines Vorratsgefäßes verbunden, daß der Inhalt des Vorratsgefäßes durch Betätigen des Applikators abgegeben werden kann.
In einer weiteren vorteilhaften Ausführungsform des Applikators kann das Vorratsgefäß dabei durch einen Deckel, welcher beim Verschließen die Öffnung des Vorratsgefäßes mit dem Applikator verdeckt, verschlossen werden. Hierbei findet die Dichtung zwischen dem Deckel und dem Fitment statt. Bevorzugt ist das Fitment dabei zumindest an der außenliegenden Seite aus einem elastischen Material hergestellt, welches bei dem verschlossenen Gefäß an der Innenseite des Deckels anliegt und hiermit die Abdichtung bewirkt. Hierzu können die Innenseite des Deckels und/oder die Außenseite des Fitments vorteilhaft mit einer Schulter oder dergleichen, welche die Dichtwirkung verstärkt, versehen sein.
Sehr bevorzugt ist die Innenfläche des Deckels dabei so ausgeformt, daß sie die Form des Fitments mit dem sich darin befindlichen Auftragskörper aufnimmt. Bei dem verschlossenen System ist dann der Raum zwischen dem Applikator und der Deckelinnenseite sehr klein.

In den üblichen Ausführungsformen des erfinderischen Applikators ist der Auftragskörper frei in alle Raumrichtungen drehbar in der Lagerung eingepaßt, insbesondere gilt dies für die rotationssymmetrischen Auftragskörper. In einer weiteren vorteilhaften Ausführungsform ist der Auftragskörper zusätzlich auf zumindest einer Drehachse gelagert. Dieses gibt der Lagerung zusätzliche Stabilität und verhindert weiterhin, daß der Auftragskörper aus dem Applikator fallen kann. Zudem ist es so möglich, die Drehrichtung des Körpers vorzugeben, was zusätzliche Möglichkeiten in der Gestaltung der Oberflächengeometrie ermöglicht. Durch achsensymmetrisch angeordnete Oberflächenstrukturen lassen sich die Transporteigenschaften der Auftragsvorrichtung weiter optimieren.
Dabei kann sowohl die Achse in ihrem Achsenlager als auch der rotationsfähige Auftragskörper auf der Achse drehbar ausgeführt sein.

Mit dem erfindungsgemäßen Applikator können flüssige und fließfähige Stoffe sowie leicht verteilbare feste Stoffe sowie Mischungen aus zwei oder mehreren Komponenten aufgetragen werden, wobei der Applikator auch hervorragend geeignet ist für Emulsionen, Suspensionen, Dispersionen, Lösungen (gasförmiger, flüssiger und fester Stoffe), Kolloide und dergleichen.
Sehr bevorzugt werden die erfinderischen Applikatoren zum Auftragen von kosmetischen oder dermatologischen Mitteln auf die Haut verwendet, insbesondere von Gelen, Emulsionen, Pickering-Emulsionen, Hydrodispersionen, Lipodispersionen.

Im technischen Sinne werden unter Gelen verstanden: Relativ formbeständige, leicht verformbare disperse Systeme aus zumindest zwei Komponenten, welche in der Regel aus einem - meist festen - kolloid zerteilten Stoff aus langkettigen Molekülgruppierungen (z.B. Gelatine, Kieselsäure, Polysaccharide) als Gerüstbildner und einem flüssigen Dispersionsmittel (z.B. Wasser) bestehen. Der kolloidal zerteilte Stoff wird oft als Verdickungs- oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können. Das Dispersionsmittel, welches das Netzwerk umgibt, zeichnet sich durch elektrostatische Affinität zum Geliermittel aus, d.h., ein vorwiegend polares (insbesondere: hydrophiles) Geliermittel geliert vorzugsweise ein polares Dispersionsmittel (insbesondere: Wasser), wohingegen ein vorwiegend unpolares Geliermittel vorzugsweise unpolare Dispersionsmittel geliert.

Starke elektrostatische Wechselwirkungen, welche beispielsweise in Wasserstoffbrückenbindungen zwischen Geliermittel und Dispersionsmittel, aber auch zwischen Dispersionsmittelmolekülen untereinander verwirklicht sind, können zu starker Vernetzung auch des Dispersionsmittels führen. Hydrogele können zu fast 100 % aus Wasser bestehen (neben beispielsweise ca. 0,2 - 1,0 % eines Geliermittels) und dabei durchaus feste Konsistenz besitzen. Der Wasseranteil liegt dabei in eisähnlichen Strukturelementen vor.

In der kosmetischen und pharmazeutischen Galenik sind ferner auch Lipogele und Oleogele (aus Wachsen, Fetten und fetten Ölen) sowie Carbogele (aus Paraffin oder Petrolatum) geläufig. In der Praxis unterscheidet man Oleogele, welche praktisch wasserfrei vorliegen, Hydrogele, welche praktisch fettfrei sind. Meistens sind Gele durchsichtig. In der kosmetischen bzw. pharmazeutischen Galenik zeichnen sich Gele in aller Regel durch halbfeste, oft fließfähige Konsistenz aus.

Ferner sind sogenannte Tensidgele gebräuchliche Zubereitungen des Standes der Technik. Darunter versteht man Systeme, die neben Wasser eine hohe Konzentration an Emulgatoren aufweisen, typischerweise mehr als ca. 25 Gew.-%, bezogen auf die Gesamtzusammensetzung. Solubilisiert man in diese Tensidgele, fachsprachlich auch "surfactant gels" genannt, Ölkomponenten, werden Mikroemulsionsgele erhalten, welche auch als "ringing gels" bezeichnet werden. Durch Zusatz von nichtionischen Emulgatoren, beispielsweise Alkylpolyglycosiden, lassen sich kosmetisch elegantere Mikroemulsionsgele erhalten.

Emulsionen sind metastabile Zwei- oder Mehrphasensysteme, bei welchen die einzelnen Phasen im flüssigen Zustand vorliegen. Die gängigsten Emulsionen sind O/W- und W/O-Emulsionen. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Die Tröpfchendurchmesser der gewöhnlichen Emulsionen liegen im Bereich von ca. 1 um bis ca. 50 µm. Solche "Makroemulsionen" sind, ohne weitere färbende Zusätze, milchigweiß gefärbt und opak. Feinere "Makroemulsionen", deren Tröpfchendurchmesser im Bereich von ca. 10⁻¹ µm bis ca. 1 µm liegen, sind, wiederum ohne färbende Zusätze, bläulichweiß gefärbt und undurchsichtig.

Mizellaren und molekularen Lösungen mit Partikeldurchmessern kleiner als ca. 10⁻² µm erscheinen klar und transparent.

Der Tröpfchendurchmesser von transparenten bzw. transluzenten Mikroemulsionen dagegen liegt im Bereich von etwa 10⁻² um bis etwa 10⁻¹ µm. Solche Mikroemulsionen sind meist niedrigviskos. Die Viskosität vieler Mikroemulsionen vom O/W-Typ ist vergleichbar mit der des Wassers.

Den bei weitem wichtigsten Produkttyp im Bereich der Hautpflegemittel beziehungsweise im Bereich kosmetischer und/oder dermatologischer Zubereitungen stellen Emulsionen dar. Emulsionen sind disperse Zwei- oder Mehrphasensysteme, wobei kosmetische Emulsionen aus mindestens einer Fettphase (Fette und mineralische Öle, Fettsäureester, Fettalkohole etc.) und mindestens einer Wasserphase (Wasser, Glycerin, Glykole usw.) bestehen, die mit Hilfe von Emulgatoren in Form feinster Tröpfchen ineinander verteilt werden. Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Die Ölphase wird vorteilhaft gewählt aus der Gruppe der Ester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren und gesättigten und/oder ungesättigten, verzweigten und/oder ungesättigten Alkoholen, aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride. Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.
Vorteilhaft kann die Ölphase einen Gehalt an cyclischen oder linearen Silikonölen, beispielsweise Cyclomethicon (Octamethylcyclotetrasiloxan), aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden. Die hier und im folgenden beschriebenen Emulsionen lassen sich also entsprechend unter teilweiser oder vollständiger Verwendung von Silikonölen als Silikonemulsionen fertigen. Entsprechendes gilt für die übrigen ölhaltigen Zubereitungen.

Dem Fachmann ist eine Vielzahl von Möglichkeiten bekannt, stabile O/W-Zubereitungen zur kosmetischen oder dermatologischen Anwendung zu formulieren, beispielsweise in Form von Cremes und Salben, die im Bereich von Raum- bis Hauttemperatur streichfähig sind, oder als Lotionen und Milche, die in diesem Temperaturbereich eher fließfähig sind und sich besonders günstig mit dem erfindungsgemäßen Applikator auftragen lassen.

Die Stabilität von Emulsionen ist unter anderem von ihrer Viskosität, insbesondere von der Viskosität der äußeren Phase abhängig. Eine Emulsion wird dann instabil, wenn sich die feindispergierten Teilchen wieder zu größeren Aggregaten zusammenballen und die sich berührenden Tröpfchen zusammenfließen. Dieser Vorgang wird als Koaleszenz bezeichnet. Der Prozeß der Koaleszenz läuft desto langsamer ab, je viskoser die äußere Phase der Emulsion ist.

O/W-Emulsionen werden dementsprechend in der Regel durch Verdickungsmittel, welche die Viskosität der wäßrigen Phase erhöhen, stabilisiert. Hierzu eignen sich beispielsweise Polyacrylate (Carbomer) und weitere organische Verdickungsmittel. Ein Nachteil dieser Methode der Stabilitätsverbesserung ist die Empfindlichkeit dieser Formulierungen gegen Elektrolyte. Ferner sind auf diese Weise naturgemäß vornehmlich höherviskose Formulierungen (wie Cremes oder Salben) herzustellen.
Der erfindungsgemäße Applikator ist aufgrund seiner besonderen Struktur hervorragend geeignet, insbesondere derart verdickte Emulsionen gleichmäßig abzugeben.

Emulsionen von "flüssiger" (= fließfähiger) Konsistenz finden in der Kosmetik beispielsweise als Deodorantien oder Antitranspirantien, als Pflege-, Reinigungs-, Gesichts- oder Handlotion Verwendung. Sie haben in der Regel eine Viskosität von etwa 50 mPa·s bis zu etwa 5000 mPa·s. Der Stabilität von fließfähigen Emulsionen ist besondere Aufmerksamkeit zu widmen, da die erheblich größere Beweglichkeit der Teilchen eine schnellere Koaleszenz fördert.

Herkömmliche Emulgatoren können entsprechend ihrem hydrophilen Molekülteil in ionische (anionische, kationische und amphotere) und nichtionische untergliedert werden:
- Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.
- Wichtige Vertreter der kationischen Emulgatoren sind die quartären Ammonium-Verbindungen.
- Der hydrophile Molekülteil nichtionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.

Durch Variation der Struktur und der Größe des polaren und des unpolaren Molekülteils lassen sich Lipophilie und Hydrophilie von Emulgatoren in weiten Grenzen verändem.

Entscheidend für die Stabilität einer Emulsion ist die richtige Auswahl der Emulgatoren. Dabei sind die Charakteristiken aller im System enthaltenen Stoffe zu berücksichtigen. Betrachtet man z. B. Hautpflegeemulsionen, so führen polare Ölkomponenten und beispielsweise UV-Filter zu Instabilitäten. Neben den Emulgatoren werden daher noch andere Stabilisatoren verwendet, die beispielsweise die Viskosität der Emulsion erhöhen und/oder als Schutzkolloid wirken.

An sich ist die Verwendung der üblichen Emulgatoren in kosmetischen oder dermatologischen Zubereitungen unbedenklich. Dennoch können Emulgatoren, wie letztlich jede chemische Substanz, im Einzelfalle allergische oder auf Überempfindlichkeit des Anwenders beruhende Reaktionen hervorrufen. Es hat daher nicht an Versuchen gefehlt, die Menge an üblichen Emulgatoren auf ein Minimum, im Idealfall sogar vollständig zu reduzieren.

Eine Reduktion der benötigten Emulgatormenge kann z. B. erreicht werden, wenn ausgenutzt wird, daß feinstverteilte Feststoffteilchen eine zusätzlich stabilisierende Wirkung haben. Dabei kommt es zu einer Anreicherung des festen Stoffes an der Phasengrenze Öl/Wasser in Form einer Schicht, wodurch Zusammenfließen der dispersen Phasen verhindert wird. Von wesentlicher Bedeutung sind dabei nicht die chemischen, sondern die Oberflächeneigenschaften der Feststoffpartikel.

Eine relativ neue technische Entwicklung ist es, kosmetische oder dermatologische Zubereitungen nur durch feinstverteilte Feststoffteilchen zu stabilisieren. Solche "emulgatorfreien" Emulsionen werden nach ihrem Erfinder auch als Pickering-Emulsionen bezeichnet. Eine Möglichkeit, eine Feststoffstabilisierung in einer kosmetischen oder dermatologischen Zubereitung vorzunehmen, ist nach May-Alert (*Pharmazie in unserer Zeit, 15*. *Jahrg*. *1986*, *Nr*. *1*, *1-7*) beispielsweise, Emulgatorgemische zu verwenden, die sowohl anionische als auch kationische Tenside enthalten. Da beim Zusammengeben von Anion- und Kationtensiden immer unlösliche, elektroneutrale Verbindungen ausfallen, läßt sich durch gezieltes Ausfällen dieser neutralen Tenside in der Grenzfläche Öl/Wasser eine zusätzliche Feststoffstabilisierung im Sinne einer Pickering-Emulsion erreichen.

Ferner beschreibt die WO-Schrift WO-98/42301 emulgatorfreie feindisperse Systeme vom Typ Wasser-in-ÖI, welche durch den Zusatz mikronisierter, anorganischer Pigmente stabilisiert werden, die aus der Gruppe der Metalloxide, insbesondere Titandioxid gewählt werden.

Emulgatorfreie Präparate auf Basis sogenannter Hydrodispersionen sind seit einiger Zeit für den Verbraucher zugängig. Hydrodispersionen stellen Dispersionen einer flüssigen, halbfesten oder festen inneren (diskontinuierlichen) Lipidphase in einer äußeren wäßrigen (kontinuierlichen) Phase dar.

Im Gegensatze zu O/W-Emulsionen, die sich durch eine ähnliche Phasenanordnung auszeichnen, sind Hydrodispersionen aber im wesentlichen frei von Emulgatoren. Hydrodispersionen stellen wie Emulsionen metastabile Systeme dar und sind geneigt, in einen Zustand zweier in sich zusammenhängender diskreter Phasen überzugehen. In Emulsionen verhindert die Wahl eines geeigneten Emulgators die Phasentrennung.

Bei Hydrodispersionen einer flüssigen Lipidphase in einer äußeren wäßrigen Phase kann die Stabilität eines solchen Systems beispielsweise dadurch gewährleistet werden, daß in der wäßrigen Phase ein Gelgerüst aufgebaut wird, in welchem die Lipidtröpfchen stabil suspendiert sind.

W/O-Lipodispersionen sind in umgekehrter Analogie emulgatorfreie feindisperse Zubereitungen vom Typ Wasser-in-Öl.

Stellen die vorstehend beschriebenen Zubereitungen die Basis für kosmetische Desodorantien/Antitranspirantien dar, so können alle gängigen Wirkstoffe vorteilhaft genutzt werden, beispielsweise Geruchsüberdecker wie die gängigen Parfümbestandteile, Geruchsabsorber (beispielsweise Schichtsilikate, femer Zinksalze der Ricinolsäure), keimhemmende Mittel. Weiterhin können vorteilhaft die üblichen Antitranspiranswirkstoffe zugegeben sein, insbesondere Adstringentien, beispielsweise basische Aluminiumchloridhydrate. Als antitranspirante Wirkstoffe sind Aluminiumsalze seit langem bekannt. Aluminiumchlorid, Aluminiumchlorhydrat, -nitrat, -sulfat, -acetat usw. werden seit einigen Jahren als antitranspirante oder antiperspirante Wirkstoffe verwendet. Daneben finden auch Zink-, Magnesium- und Zirkoniumverbindungen Anwendung. Der Stand der Technik wird beispielsweise beschrieben in: *H. P. Fiedler*, *Der Schweiß, Editio Cantor, Aulendorf*, *2*. *Auflage, S*. *303-377*, *Kapitel K: "Mittel zur Hemmung der Transpiration".* Von den dort als Antitranspirant-Wirkstoffe beschriebenen Metallverbindungen haben sich für die Anwendung in kosmetischen und dermatologischen Antitranspirantien nur die Aluminiumsalze und - in etwas geringerem Maße - die Aluminium/Zirkoniumsalze durchgesetzt. Diese können hier ebenso wie auch andere Metall- und Nichtmetall-Antitranspirant-Wirkstoffe eingesetzt werden.

Einige Beispiele von Desodoratien bzw. Antitranspirantien in Form von Gelen und Emulsionen, für welche der erfinderische Applikator hervorragend Anwendung finden kann, sind in den folgenden zwei Tabellen zusammengestellt.

| | | Gel 1 | Gel 2 | Gel 3 |
|---|---|---|---|---|
| (a) | 1,3-Butylenglycol | 2,00 | 2,00 | 2,00 |
| | Hydroxyethylcellulose | 0,50 | 0,50 | 0,50 |
| (b) | Ethylalkohol | 60,00 | 60,00 | 60,00 |
| | PEG-40-Hydriertes Ricinusöl | 2,00 | 2,00 | 2,00 |
| | Diglycerinmonolaurat | 0,30 | | |
| | Triglycerinmonolaurat | | 0,30 | |
| | Diglycerinmonocaprinat | | | 0,30 |
| | Parfum | q.s. | q.s. | q.s. |
| (c) | Wasser | ad 100,00 | ad 100,00 | ad 100,00 |
| Alle Angaben in Gew.-% | | | | |

Die unter (a) genannten Bestandteile werden dispergiert. Wasser (c) wird zugegeben, bei Raumtemperatur quellen gelassen, eine Lösung der unter (b) genannten Bestandteile wird nach ca. 15 Minuten zugegeben. Die entstandene Mischung wird homogenisiert und kann abgefüllt werden.

| | | Emulsion 1 | Emulsion 2 | Emulsion 3 |
|---|---|---|---|---|
| (a) | Tricetearethphosphat | 0,30 | 0,30 | 0,30 |
| | Octyldodecanol | 2,00 | 2,00 | 2,00 |
| | C₁₂₋₁₅-Alkohol-Benzoate | 2,00 | 2,00 | 2,00 |
| | Diglycerinmonolaurat | 0,50 | | |
| | Triglycerinmonolaurat | | 0,50 | |
| | Diglycerinmonocaprinat | | | 0,50 |
| | C₁₀₋₃₀-Alkylacrylate | 0,15 | 0,15 | 0,15 |
| (b) | Ethylalkohol | 10,00 | 10,00 | 10,00 |
| | Parfum | q.s. | q.s. | q.s. |
| (c) | NaOH | 0,05 | 0,05 | 0,05 |
| | Wasser | ad 100,00 | ad 100,00 | ad 100,00 |
| Alle Angaben in Gew.-% | | | | |

Die unter (a) und (c) genannten Bestandteile werden jeweils unter Rühren auf 75 °C erwärmt. Sodann werden die Bestandteile (a) zu (c) gegeben. Die Mischung wird auf 35 °C abgekühlt. Aus den Bestandteile (b) wird eine Lösung hergestellt, welche auf 35 °C erwärmt und unter Rühren zur Mischung aus (a) und (c) gegeben wird.

Eine weitere Antitranspirant-Zubereitung, für welche der erfinderischen Applikator besonders gut geeignet ist, setzt sich gemäß der folgenden Aufstellung zusammen:

| | | |
|---|---|---|
| a | Isosorbidmonolaurat | 4 |
| | Polypropylenglycol-15 Stearylether | 2 |
| | Polyethylenglycol-25 Stearylether | 2 |
| | Polyethylenglycol-3 Stearylether | 2 |
| | Glycerylmonolaurat | 1 |
| b | Glycerin | 5 |
| | Polyethylenglycol-10 | 1 |
| | Wasser | ad 100 |
| c | Cyclomethicon | 1 |
| d | Parfum | q.s. |
| e | Aluminumchlorohydrat | 40 |
| Alle Angaben in Gew.-% | | |

Die unter (a) und (b) genannten Bestandteile werden jeweils unter Rühren auf 70 °C erwärmt. Sodann werden die Bestandteile (b) zu (a) gegeben. Die Mischung wird auf 45 °C abgekühlt, sodann Bestandteil (c) addiert. Nach Abkühlung auf 35 °C wird Bestandteil (d) dazugegeben, bei Erreichen von 30 °C schließlich Bestandteil (e). Bei Bedarf kann die so hergestellte Emulsion auch homogenisiert werden.

Für alle im vorderen beschriebenen Zubereitungen und Mittel ist der erfindungsgemäße Applikator hervorragend geeignet und stellt gegenüber dem Stand der Technik einen wesentliche Vorteile dar. Besonders empfindliche Mehrphasensysteme werden in den Vertiefungen des Auftragskörpers des erfinderischen Applikators weniger Beanspruchungen ausgesetzt als auf den herkömmlichen makroskopisch glatten Auftragskörpem. Auch Dispersionen, hier insbesondere Fest-in-flüssig-Systeme, lassen sich mit dem erfindungsgemäßen Applikator besser auftragen, da die festen Stoffe in den Vertiefungen des Auftragskörpers vorteilhafter auf die Abgabeseite des Applikators transportiert werden können.

Diese und weitere kosmetische und dermatologische Zubereitungen können zusammen mit dem erfinderischen Applikator sehr vorteilhaft in Form von Deo- und/oder Antitranspirantstiften, Lippenpflegestiften, medizinischen oder dermatologischen Behandlungsstiften bei Hautkrankheiten, Abdeckstifte für Hautunreinheiten, Stifte im Bereich der dekorativen Kosmetik (beispielsweise dekorative Lippenstifte oder Kajalstifte), um nur einige Beispiele aus dem sehr breiten Feld der Anwendungen zu erwähnen.

Teil der Erfindung ist somit auch die Verwendung eines rotationsfähigen geometrischen Körpers mit einer derart strukturierten Oberfläche, daß durch die Strukturierung insbesondere Vertiefungen in der Oberfläche gebildet werden, zur Applikation von Substanzen auf ein Substrat, bevorzugt zur Applikation von kosmetischen oder dermatologischen Zubereitungen, insbesondere von Deodorantien und/oder Antitranspirantien, sehr bevorzugt von Al- und/oder Al/Zr-Komplexe enthaltenden Deodorantien und/oder Antitranspirantien, auf die Haut.

Dabei können die kosmetischen oder dermatologischen Zubereitungen vorteilhaft in Form von Gelen, Emulsionen, Mikroemulsionen, Suspensionen, Dispersionen, Kolloiden, Pudern, Pulvem und/oder Pasten vorliegen.

Teil der Erfindung ist aber auch die Verwendung des im vorderen beschriebenen Applikators, welcher durch einen rotationsfähigen geometrischen Körper gekennzeichnet ist, zur Applikation von Substanzen auf ein Substrat, insbesondere in einer oder mehrerer im vorderen beschriebenen Einzelheiten. Hierzu sind alle beschriebenen Ausführungsformen des erfinderischen Applikators hervorragend geeignet.

Gleichwohl bietet sich die Verwendung des erfinderischen Applikators aber auch überall dort an, wo es von Interesse sein kann, Flüssigkeiten punkt- oder strichgenau aufzutragen, beispielsweise im Bereich der Flüssigklebstoffe, der pharmazeutischen Mittel, der Reinigungsmittel (beispielsweise für Fleckentfemer), der Lebensmittel und dergleichen.
Auch die Verwendung als Tintenauftragskugel in Kugelschreibern und anderen Schreibstiften bietet sich für den erfinderischen Applikator an. Besonders für höherviskose Schreibflüssigkeiten, beispielsweise für die Lackfarbe in Lackstiften, ist ein strukturierter Auftragskörper besonders gut geeignet.

Es soll nicht unerwähnt bleiben, daß der erfinderische Applikator sich aufgrund der Strukturierung der Auftragskugel in besonders guter Weise eignet, gerade hochvikose, fettartige Substanzen zu transportieren, für die herkömmliche Applikatoren nicht oder nur sehr schlecht eignen würden. Ein Beispiel hierfür ist die bereits vorstehend erwähnte Verwendung für dekorative Lippenstifte, deren Zusammensetzung im allgemeinen auf Fettbasis beruht.

Wird der erfinderische Applikator entsprechend dimensioniert, so läßt er sich auch zum Auftragen von Flüssigkeitsfilmen auf größere Flächen verwenden. Hier lassen sich zum Beispiel Applikatoren, bei denen der rotationsfähige Auftragskörper aus einer entsprechend strukturierten Walze besteht, zum Auftragen von Wandfarbe für Dekorations- und Renovierungszwecke oder zum Auftragen von Schutzfilmen verwenden. Bevorzugt ist die Walze dabei zusätzlich aus einem saugfähigen Material. Ein Vorteil zu den bisherigen, auf einer freien Achse gelagerten Farbrollen aus fellartigem Material ergibt sich daraus, daß das Applikatorsystem sein Farbvorratsgefäß bereits mitbringt, so daß wesentlich sauberer als bisher gearbeitet werden kann. Statt die Farbwalze jeweils erneut in den Farbeimer tauchen zu müssen, kann der Applikator durch ein geeignetes Adaptersystem, beispielsweise durch eine Schraubvorrichtung, auf ein neu gefülltes Vorratsgefäß aufgesetzt werden.
Gleichzeitig kann die Auftragsvorrichtung derart gestaltet werden, daß der Reinigungsvorgang - im Vergleich zu Fellrollen, welche umständlich ausgewaschen werden müssen - erheblich erleichtert wird, da beispielsweise ein einfaches Abspülen zur Reinigung ausreicht.

Die Ausmaße des erfindungsgemäßen Applikators können jeweils der gewünschten Funktion bzw. Verwendung angepaßt werden. So werden für eine Auftragsvorrichtung für Deodorant- und/oder Antitranspirant-Roller besonders bevorzugt Kugeln mit einem Durchmesser von 10 bis 50 mm, insbesondere von 20 bis 35 mm, sehr bevorzugt von 28 bis 30 mm verwendet. In einer der hier bevorzugten Varianten liegen runde muldenförmige Vertiefungen, jeweils mit einem Durchmesser von 2 bis 5 mm vor.
Für die Verwendung als Applikator für Lippenstifte kann man bevorzugt Kugeln mit einem Durchmesser von 5 bis 20 mm verwenden.

Neben den bereits geschilderten Vorteilen bezüglich der Auftrageeigenschaften für Flüssigkeiten besitzen die erfindungsgemäßen Applikatoren im Bereich der Körperpflege aufgrund des durch die veränderte Oberfläche hervorgerufenen Massageeffektes einen zusätzlichen Vorteil für den Anwender. Während des Auftragens von kosmetischen oder dermatologischen Zubereitungen kann gleichzeitig ein positiver Effekt beispielsweise für die Hautstraffung oder gegen Cellulitis erzielt werden.

Der Auftragskörper kann aus Metall, Glas, Keramik, Porzellan oder einem geeigneten Kunststoff bestehen. Sehr günstig ist es, als Material Polypropylen zu verwenden. Gerade für die Verwendung als Applikator für Klebstoffe bieten sich Materialien an, die sich nicht oder schlecht verkleben lassen, insbesondere Teflon.
Hervorragend geeignet sind allgemein Thermoplaste, Elastomere oder Kombinationen von Kunststoffen aus diesen Gruppen. Ihre Eigenschaften lassen sich durch Zusätze von Weichmachern, Füllstoffen, Stabilisatoren und anderen Additiven sowie durch Faserverstärkung breit variieren. Als Beispiele für thermoplastische und elastische Kunststoffe seien genannt: alle aus linearen oder thermolabil vemetzten Polymer-Molekülen bestehenden Kunststoffe, z. B. Polyolefine, Vinylpolymere, Polyamide, Polyester, Polyacetale, Polycarbonate, z. T. auch Polyurethane u. lonomere; TPE-S (Styrol-Oligoblock-Copolymere), TPE-O (thermoplastische Polyolefine), TPE-U (thermoplastische Polyurethane), TPE-E (entsprechende Copolyester), TPE-A (entsprechende Copolyamide), Natur- und Synthesekautschuke.

Es kann für entsprechende Verwendungen vorteilhaft sein, die Auftragsvorrichtung aus einem saugfähigen Material herzustellen oder aus einem nicht saugfähigen Kern, welcher mit einer saugfähigen Schicht umgeben ist. Durch die Dicke dieser Schicht läßt sich die jeweils abgegebene Menge zusätzlich dosieren: Dicke saugfähige Schichten können große Flüssigkeitsmengen pro "Rollvorgang" aufnehmen und wieder abgeben, dünne saugfähige Schichten eignen sich entsprechend für einen sparsamen Auftragsvorgang.

Durch die mit einem saugfähigen Material umschichtete Auftragsvorrichtung ist bereits ein Beispiel für Auftragsvorrichtungen gegeben, welche aus mehr als einem Material bestehen. Solche Auftragsvorrichtungen können beispielsweise auch im Zweikomponentenspritzguß aus zwei oder mehreren verschiedenen oder aus zwei oder mehreren unterschiedlich gefärbten bzw. eingefärbten Materialien hergestellt werden.
Auftragsvorrichtungen, welche zum Beispiel einen dunklen "Kern" und eine helle "Hülle" haben, welche die Vertiefungen aufweist, haben im trockenen, nicht gefüllten Zustand ein Erscheinungsbild, welches dunkle Flecken (Kreise oder dergleichen) dort in einen hellen Umfeld zeigt, wo sich die Vertiefungen befinden. Werden dies jetzt mit einer hellen Flüssigkeit, beispielsweise mit weißer Creme, gefüllt, so läßt sich die Füllmenge und der Benetzungsgrad der Auftragsvorrichtung optisch wahrnehmen. Hierdurch kann die aufzutragende Menge besser dosiert werden.
Unterschiedlich gefärbte dekorative Vertiefungen beispielsweise können die Werbefunktion erhöhnen. So lassen sich dekorative Vertiefungen bzw. Erhebungen beispielsweise in Form von Kleeblättern, Comicfiguren oder anderen Ornamenten gestalten, wobei entweder die Vertiefungen eine andere Färbung besitzen als die Oberfläche der erhobenen Bereiche, weiterhin können die einzelnen Vertiefungen oder Erhebungen jeweils unterschiedlich gefärbt sein.

Der erfinderische Applikator soll im folgenden durch einige Abbildungen veranschaulicht werden, ohne sich durch die Wahl der abgebildeten Beispiele unnötig beschränken zu wollen. Es zeigen:
Figur 1 bis 4 unterschiedliche Ausführungsformen des Auftragskörpers
Figur 5 eine schematische Zeichnung des Applikators
Figur 6 eine schematische Zeichnung der Lagerung und der Abdichtung
Figur 7 einen Einblick von oben in ein beispielhaftes Fitment

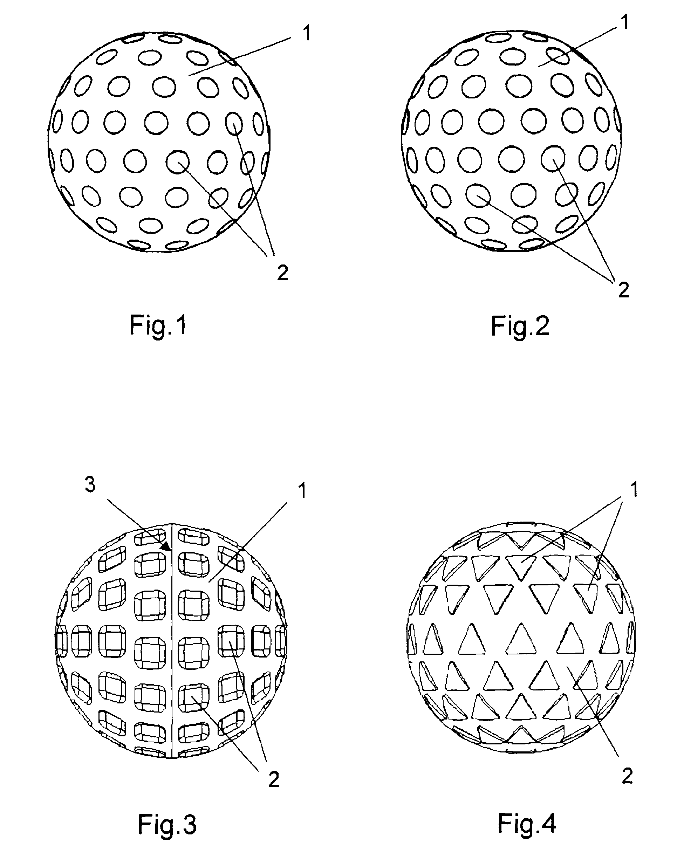

In den Figuren 1 bis 4 sind beispielhaft vier unterschiedliche Ausführungsformen des Auftragskörpers, hier jeweils in Form einer Kugel, dargestellt. Dargestellt ist jeweils die Kugelebene (1), welche als makroskopische Oberfläche der Kugel angesehen werden kann, sowie die Vertiefungen (2) in dieser Ebene.
In den Figuren 1 bis 3 liegen diese Vertiefungen (2) separat voneinander vor, und zwar in Form von kreisförmigen Mulden [Figuren 1 und 2, unterschiedliche Anordnungen der Vertiefungen (2)] beziehungsweise in Form von quadratischen Vertiefungen [Figur 3]. In Figur 3 ist außerdem die Nahtlinie (3) dargestellt, welche durch den Spritzguß in einer zweiteiligen Gußform oder bei Aufbau der Kugel aus zwei Kugelhälften entsteht.

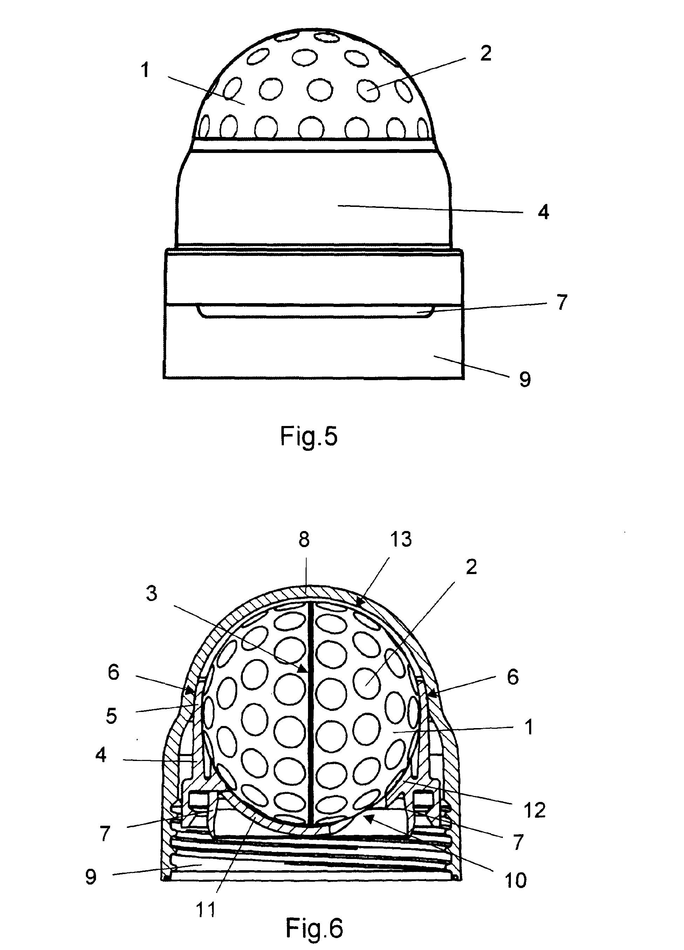

Figur 4 zeigt einen kugelförmigen Auftragskörper mit einem System aus kanalartigen, verbundenen Vertiefungen (2) in der Oberfläche (1) ["offenporiges System"]. Dieses System kann auch derart betrachtet werden, daß die (in diesem Beispiel dreieckförmigen) Bereiche (1) Erhebungen auf der durch die "Kanalebene" (2) gebildeten Kugel darstellen. Figur 5 zeigt einen Applikator mit einem durch Vertiefungen (2) strukturierten Auftragskörper (1), welche derart in eine Lagerung (4) eingepaßt ist, daß sie etwa hälftig aus dieser herausragt. Mit einer Einsteckhülse (7) kann der Applikator beispielsweise in der Öffnung eines Vorratsgefäßes (9) verankert sein.

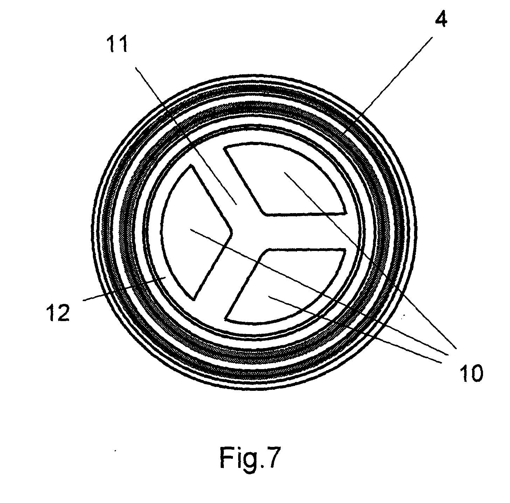

In Figur 6 ist ein Schnitt durch einen Applikator, welcher aus einer Lagerung (4) und einem Auftragskörper (1) besteht, dargestellt. Der Auftragskörper (1) besteht hier aus einer Kugel mit runden Vertiefungen (2), die Positionsziffer (3) verweist wieder auf eine herstellungsbedingte Nahtstelle. Die Lagerung (4) besteht aus einem röhrenförmigen Fitment (5), in welches der Auftragskörper (1) eingesetzt werden kann, und einem ringförmigen Auflagebett (12) mit stabilisierenden in das Ringinnere verlaufenden Streben (11) [vgl. hierzu auch Figur 7]. Vorteilhaft verjüngt sich das Fitment (4) nach oben hin, damit der Auftragskörper (1) nicht herausfallen kann.
Durch die Öffnungen (10) im Auflagebett wird der Auftragskörper mit der aufzutragenden Substanz aus dem darunter liegenden Vorratsgefäß (9) benetzt, von diesem ist schematisch das Schraubgewinde dargestellt. Die Verankerung des Applikators in dem Vorratsgefäß (9) kann beispielsweise durch eine Einsteckhülse (7) geschehen.

Das Vorratsgefäß kann durch einen Deckel (8) verschlossen werden, welcher in einer günstigen Ausführungsform die Form des Auftragskörpers aufnimmt. Der Raum (13) zwischen dem Auftragskörper und dem Deckel ist dann klein. Ist das Fitment (5) aus einem hinreichend flexiblen oder weichen Material hergestellt, so findet eine Abdichtung des verschlossenen Vorratsgefäßes in hervorragender Weise im Bereich der Positionsziffer (6) statt.
Figur 7 zeigt einen Einblick von oben in ein beispielhaftes Fitment (4), bei dem das Auflagebett (12) für den Auftragskörper ringförmig gestaltet ist. Zur Stabilisierung und zur besseren Halterung der Kugel sind in dieser Ausführungsform stemartig Streben (11) angebracht. Die Öffnungen (10) erlauben eine Benetzung mit der aufzutragenden Substanz.

## Patentansprüche

1. Applikator für Flüssigkeiten, fließfähige Formulierungen, Pasten, Pulver und dergleichen, bestehend aus einer Lagerung und einem in der Lagerung gehaltenen, rotationsfähigen geometrischen Körper, wobei der rotationsfähige geometrische Körper zumindest mit einem Teil seiner Oberfläche freiliegt, **dadurch gekennzeichnet, daß**
der rotationsfähige geometrische Körper (1) eine strukturierte Oberfläche aufweist.

2. Applikator nach Anspruch 1, **dadurch gekennzeichnet, daß**
es sich bei den fließfähigen Formulierungen um Emulsionen, Suspensionen, Kolloide, Dispersionen, Gele oder Lösungen handelt.

3. Applikator nach zumindest einem der vorangehenden Ansprüche, **dadurch gekennzeichnet, daß**
der rotationsfähige Körper ein rotationssymmetrischer geometrischer Körper ist.

4. Applikator nach zumindest einem der vorangehenden Ansprüche, **dadurch gekennzeichnet, daß**
durch die Strukturierung Vertiefungen (2) in der Oberfläche des rotationsfähigen Körpers (1) gebildet werden, insbesondere Vertiefungen (2) in symmetrischer Anordnung und/oder insbesondere Vertiefungen (2) in Form von Mulden, Rinnen, Kerben und dergleichen.

5. Applikator nach Anspruch 1, **dadurch gekennzeichnet, daß** der rotationsfähige geometrische Körper (1) eine Kugel, ein Ellipsoid oder eine Walze ist.

6. Applikator nach zumindest einem der vorangehenden Ansprüche, **dadurch gekennzeichnet, daß**
der Applikator Teil eines den abzugebenden Stoff beinhaltenden Behälters (9), insbesondere eines flaschenartigen Behälters, ist.

7. Applikator nach zumindest einem der vorangehenden Ansprüche, **dadurch gekennzeichnet, daß**
die Lagerung (4) gebildet wird aus einem Halterungssitz, welcher der Form des rotationsfähigen Körpers (1) derart angepaßt ist, daß der rotationsfähige Körper (1) so in dem Halterungssitz eingepaßt ist, daß er zu einem Teil aus dem Halterungssitz herausragt

8. Applikator nach zumindest einem der vorangehenden Ansprüche, **dadurch gekennzeichnet, daß**
der rotationsfähige geometrische Körper (4) zusätzlich auf zumindest einer Drehachse gelagert ist.

9. Verwendung eines rotationsfähigen geometrischen Körpers (1) mit einer derart strukturierten Oberfläche, daß durch die Strukturierung insbesondere Vertiefungen (2) in der Oberfläche gebildet werden, zur Applikation von Substanzen auf ein Substrat, bevorzugt zur Applikation von kosmetischen oder dermatologischen Zubereitungen, insbesondere von Deodorantien und/oder Antitranspirantien, sehr bevorzugt von Al- und/oder Al/Zr-Komplexe enthaltende Deodorantien und/oder Antitranspirantien, auf die Haut.

10. Verwendung nach Anspruch 9, **dadurch gekennzeichnet, daß**
die kosmetischen oder dermatologischen Zubereitungen in Form von Gelen, Emulsionen, Mikroemulsionen, Suspensionen, Dispersionen, Kolloiden, Pudern, Pulvem und/oder Pasten vorliegen.
